(19) Europäisches Patentamt
European Patent Office
Office européen des brevets

(11) Publication number: 0 204 948
B1

(12) EUROPEAN PATENT SPECIFICATION

(45) Date of publication of patent specification: 28.03.90

(51) Int. Cl.$^5$: A 23 K 1/16, A 61 K 31/135

(21) Application number: 86106094.5

(22) Date of filing: 30.01.84

(60) Publication number of the earlier application in accordance with Art. 76 EPC: 0 117 647

(54) Growth promotion of animals with phenethanol amines.

(30) Priority: 31.01.83 US 462587

(43) Date of publication of application:
17.12.86 Bulletin 86/51

(45) Publication of the grant of the patent:
28.03.90 Bulletin 90/13

(84) Designated Contracting States:
BE CH DE FR IT LI LU NL SE

(56) References cited:
EP-A-0 006 766

(73) Proprietor: ELI LILLY AND COMPANY
Lilly Corporate Center
Indianapolis Indiana 46285 (US)

(72) Inventor: Anderson, David Bennett
1334 Greenhills Road
Greenfield Indiana 46140 (US)
Inventor: Schmiegel, Klaus Kurt
4507 Staughton Drive
Indianapolis Indiana 46226 (US)
Inventor: Veenhuizen, Edward Lee
R.R. 3, Box 41
Greenfield Indiana 46140 (US)
Inventor: TUTTLE, Ronald Palph
2704 Vista Del Sembrado
Escondido, California 92025 (US)

(74) Representative: Hudson, Christopher Mark et al
Erl Wood Manor
Windlesham Surrey GU20 6PH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The chemistry and use of β-phenethanolamines has been extensively investigated. European Patent Specification No. 6,766 describes the use of phenethanolamine derivatives in promoting the loss of weight in animals.

This invention provides a method for increasing weight gain in domestic animals and improving the efficiency of feed utilization and the quality of the carcass. The invention is more particularly directed to a method for promoting growth, improving feed efficiency or leanness comprising administering a compound having the formula (I)

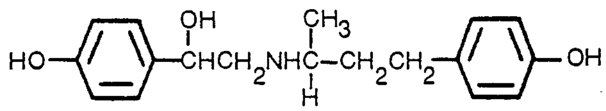

(I)

or an acid addition salt thereof. Our copending European Patent Application No. 84300559.6 (Publication No. 0 117 647) discloses the use of related compounds.

This invention also provides an animal feed premix comprising a β-phenethanolamine of the above formula together with a suitable carrier therefor.

The compound of formula (I) is disclosed as having utero-relaxing activity by Van Dijk et al. in *Recueil, 92* 1281 (1973).

The compound of formula (I) can be prepared by reaction of a styrene oxide with a 3-phenylpropylamine derivative.

An alternative method for preparing the β-phenethanolamine comprises reacting a mandelic acid derivative with a 3-phenylpropylamine derivative to provide an amide, which upon subsequent reduction provides a compound of the above formula.

A similar, yet alternative, method of synthesis comprises reacting a phenethanolamine with a phenylethyl ketone to provide a Schiff base, which upon reduction gives a compound to be employed in the present invention. Thus, 2-hydroxy-2-(4-hydroxyphenyl)ethylamine can be reacted with a ketone such as methyl 2-(4-hydroxyphenyl)ethyl ketone to provide the corresponding imine, which upon reduction, for instance with 5% palladium on carbon, provides 1-(4-hydroxyphenyl)-2-[1-methyl-3-(4-hydroxyphenyl)-propylamino]ethanol.

It should be noted that the compounds to be employed in this invention possess two asymmetric centers. Since employment of individual optical isomers necessitates preparing the β-phenethanolamines from optically active starting materials, or using costly separation procedures, a preferred embodiment of this invention for veterinary purposes employs a mixture of optical isomers. Thus, 1-(4-hydroxyphenyl)-2-[1-methyl-3-(4-hydroxyphenyl)propylamino]ethanol is preferably prepared from racemic mixtures of starting materials, e.g. dl-1-methyl-3-(4-hydroxyphenyl)propylamine and dl-4-hydroxystyrene oxide, to provide a mixture of all four possible optical isomers of the product. The mixture of optical isomers is employed in the method without subsequent separation of isomers.

Since the β-phenethanolamine to be employed in the present method is inherently basic, it readily forms acid addition salts with any number of inorganic and organic acids These salts can be empoloyed in the invention, and often are preferred to the free base since they generally are more soluble in solvents such as water and are more conveniently formulated as an animal feedstuff. Acids commonly employed to form acid addition salts include mineral acids such as hydrochloric acid, sulfuric acid, phosphoric acid, perchloric acid and the like; and organic acids such as acetic acid, citric acid, succinic acid, para-toluene sulfonic acid, methanesulfonic acid, lactic acid and the like. Preferred salts to be employed in the present method include the hydrochlorides and hydrobromides.

The method of promoting growth and improving leanness and feed efficiency provided by this invention is practised by administering an effective amount of a compound of formula (I) to a domesticated animal that receives a nutritionally adequate diet. The term "domesticated animal" as used herein includes those warm-blooded animals raised for human meat consumption, for example grower/finisher swine, poultry, ruminants and the like, but excludes such animals as rats. In a preferred embodiment, the growth of pigs, chickens and turkeys is promoted employing a β-phenethanolamine. Another preferred embodiment is practiced in ruminants such as cattle, sheep and goats. The method of improving leanness is not limited to meat producing animals, and can be practiced on other warm-blooded animals, including dogs and cats. This latter embodiment is particularly useful when it is desired to maintain mature animals in a relatively lean physical state.

The method of the invention is preferably practiced by orally administering an effective amount of a β-phenethanolamine to an animal. Other routes of administration can be employed, for instance intramuscular or intravenious injection; however, such routes are less practical. The amount to be administered to an animal is the amount that is effective in causing a promotion of growth, or an improvement in the efficiency of utilization of food, or an improvement in carcass quality of the animal, for instance in the form of less fatty tissue and improved leanness. The effective amount to be administered will vary somewhat depending upon the particular animal species being treated and the particular active ingredient employed, but generally will be from about 1 to about 1000 parts per million (ppm) of total daily

feed intake. Such amount will provide a dosage of about 0.05 to about 50 mg/kg. A preferred embodiment employs about 1 to about 200 ppm, and more preferably from about 5 to about 100 ppm. A typical amount of active ingredient to be administered to swine will be from about 5 to about 40 ppm. For example, when practicing the method in animals such as ruminants or swine, the compound will be added to the daily feed ration at about 5 to about 100 parts per million of the daily feed ration.

For oral administration, the active β-phenethanolamine is preferably admixed with suitable carriers or diluents commonly employed in animal husbandry. Animal feed premixes comprising a β-phenethanolamine as defined herein are provided as a further embodiment of this invention. Typical carriers and diluents commonly employed in such feedstuffs include corn meal, soybean meal, alfalfa meal, rice hulls, soybean mill run, cottonseed oil meal, bone meal, ground corn, corncob meal, sodium chloride, urea, cane molasses and the like. Such carriers promote a uniform distribution of the active ingredient in the finished feed ration into which such compositions are added, thereby ensuring proper distribution of the active ingredient throughout the feed. The feedstuff composition provided by the invention will contain about 5 to about 95 percent by weight of active ingredient, and more typically about 10 to about 50 percent by weight. As already noted, the acid addition salts of the active phenethanolamines are generally preferred for oral administration, and are thus the preferred form of active ingredient for the feedstuff compositions of the invention.

While the preferred method for orally administering the growth promoters is via the daily feed rations, the compounds can be incorporated into salt blocks and mineral licks, as well as being added directly to drinking water for convenient oral consumption. The compounds can additionally be formulated with polymorphous materials, waxes and the like for long-term controlled release, and administered to an animal as a bolus or tablet only as needed to maintain the desired daily payout of active ingredient.

For parenteral administration, the β-phenethanolamines can be admixed with conventional carriers such as corn oil, sesame oil, carbowax, calcium stearate and the like. Such formulations can be molded into pellets and administered as an injection or as a slow-release subcutaneous implant. Such administrations can be made as often as needed to ensure the proper dosing of active ingredient to obtain the desired rate of growth promotion and improvement in leanness and feed efficiency.

While the compounds described herein are effective in promoting average daily weight gain and improving feed efficiency in animals, they also cause observable improvement in the quality of the meat produced. For example, the compounds appear to mobilize free fatty acids from fatty tissues and depress the deposition of fat as the animals gain weight. This reduction of fat is beneficial since the animal being treated according to the invention gains weight in the form of more useable lean meat, thereby reducing waste and improving the value of the animal thus treated. Also, mature animals that are not subject to additional weight gain can be maintained in a desirably lean form by administering a compound as described herein.

The compound of formula (I) can be prepared by the following procedures.

Example 1

Preparation of dl-4-(benzyloxy)mandelic acid

A solution of 5.0 g of dl-4-hydroxy mandelic acid, 11.0 g of benzyl chloride and 10.0 g of potassium carbonate in 50 ml of methanol was heated to reflux and stirred for seventy-two hours. The reaction mixture was cooled to room temperature and diluted with 50 ml of water. The aqueous soltution was washed twice with 50 ml portions of benzene, and then was acidified with concentrated hydrochloric acid. The aqueous acid solution was extracted three times with 50 ml portions of ethyl acetate. The organic extracts were combined, washed with water and with saturated sodium chloride solution and dried. Removal of the solvent by evaporation under reduced pressure provided 5.7 g of a solid which was then recrystallized from 300 ml of toluene to afford 5.33 g of dl-4-(benzyloxy)mandelic acid. M.P. 153—155°C.

Analysis calc. for $C_{15}H_{14}O_4$

Theory: C, 69.76; H, 5.46;

Found: C, 69.96; H, 5.33.

Example 2

Resolution of dl-4-(benzyloxy)mandelic acid to provide R(−)-(4-benzyloxy)mandelic acid

To a stirred solution of 185.6 g of dl-4-benzyloxy)mandelic acid in 2500 ml of ethyl acetate at 80°C was added in one portion 43.6 g of R(+)-α-methylbenzylamine. The reaction mixture was cooled to room temperature, and the precipitated solid which had formed was collected by filtration and washed with fresh ethyl acetate. The solid was recrystallized twice from a solution of ninety percent ethanol in water to provide 91.4 g of the R(+)-α-methylbenzylamine salt of R(−)-4-(benzyloxy)mandelic acid. M.P. 208.5—209.5°C. $[\alpha]_D$ −38.6°, $[\alpha]_{365}$ −155.3° (MeOH).

Analysis calc. for $C_{23}H_{25}NO_4$

Theory: C, 72.80; H, 6.64; N, 3.69;

Found: C, 72.95; H, 6.83; N, 3.95.

To a stirred suspension of 91.4 g of the above-named salt in 2000 ml of ethyl acetate was added 150 ml of ten percent aqueous hydrochloric acid solution. The aqueous acid solution was separated, and the organic layer washed with water and dried. Removal of the solvent by evaporation under reduced pressure afforded 54.5 g of R(−)-4-(benzyloxy)mandelic acid, i.e. R(−)-2-(4-benzyloxyphenyl)-2-hydroxyacetic acid.

M.P. 155—161°C. $[\alpha]_D$ −102.2°; $[\alpha]_{365}$ −410.6° (MeOH)

Analysis calc. for $C_{15}H_{14}O_4$

Theory: C, 69.76; H, 5.46;

Found: C, 69.67; H, 5.41.

Example 3

Preparation of dl-1-methyl-3-(4-benzyloxyphenyl)propylamine

A solution of 40.0 g of methyl 2-(4-benzyloxyphenyl)ethyl ketone and 160 ml of anhydrous ammonia in 300 ml of ethanol was heated at 75°C and stirred for two hours. After cooling the reaction mixture to room temperature, 4.0 g of Raney nickel was added in one portion, and the reaction mixture then was stirred at 25°C for twelve hours under a hydrogen atmosphere at 300 psi. The reaction mixture next was filtered and the filtrate was concentrated by evaporation of the solvent under reduced pressure to provide an oil. The oil was dissolved in 120 ml of 3N hydrochloric acid, from which the product as a hydrochloride salt precipitated. The salt so formed was collected by filtration and recrystallized from methanol and toluene to provide 36.2 g of dl-1-methyl-3-(4-benzyloxyphenyl)propylaminium chloride. M.P. 195—197.5°C.

Example 4

Resolution of dl-1-methyl-3-(4-benzyloxyphenyl)propylamine to provide R-(−)-1-methyl-3-(4-benzyloxyphenyl)propylamine

A solution of 629.3 g of dl-1-methyl-3-(4-benzyloxyphenyl)propylaminium chloride was converted to the free amine by reaction with 95 g of sodium hydroxide in 400 ml of water. The free amine was then dissolved in 2000 ml of methylene chloride and added to a solution of 328 g of D-(−)-mandelic acid in 1000 ml of methanol. The mandelic acid salt of the free amine precipitated out of solution was recrystallized three times from methanol to provide 322 g of the R-mandelic acid salt of R-1-methyl-3-(4-benzyloxyphenyl)propylamine. M.P. 166—167°C. $[\alpha]_D$ −30°, $[\alpha]_{365}$ −119° (MeOH).

The salt so formed was converted to R-1-methyl-3-(4-benzyloxyphenyl)propylamine as the free base by reaction with aqueous sodium hydroxide.

Example 5

R,R-N-[2-(4-Benzyloxyphenyl)-2-hydroxy-1-oxoethyl]-1-methyl-3-(4-benzyloxyphenyl)propylamine

A solution of 93.9 g of R-1-methyl-3-(4-benzyloxyphenyl)propylamine in 500 ml of N,N-dimethylformamide containing 63.0 g of 1-hydroxybenzotriazole and 104.6 g of R-2-(4-benzyloxyphenyl)-2-hydroxyacetic acid was cooled to 0°C and stirred while a solution of 83.6 g of N,N′-dicyclohexylcarbodiimide in 300 ml of dimethylformamide was added dropwise over one hour. The reaction mixture was stirred for twelve hours at 3°C and then was diluted with 10 ml of water, stirred for an additional hour, and then cooled to −30°C in an ice-acetone bath. The reaction mixture was filtered to remove dicyclohexylurea, and then the filtrate was concentrated by evaporation of the solvent under reduced pressure. The concentrated solution was diluted with ethyl acetate and washed with saturated aqueous sodium carbonate solution, with water, with 300 ml of 1N hydrochloric acid, and again with water. The organic layer was dried and the solvent was removed by evaporation under reduced pressure to provide the product as a white solid. The solid was recrystallized once from acetonitrile and again from methanol to provide 159.7 g of R,R-N-[2-(4-benzyloxyphenyl)-2-hydroxy-1-oxoethyl]-1-methyl-3-(4-benzyloxyphenyl)propylamine. M.P. 145—148°C. $[\alpha]_D$ −15.9°, $[\alpha]_{365}$ −50.1° (MeOH).

Analysis calc. for $C_{32}H_{33}NO_4$

Theory: C, 77.55; H, 6.71; N. 2.83;

Found: C, 77.04; H, 6.84; N, 2.53.

Example 6

R,R-N-[2-(4-Benzyloxyphenyl)-2-hydroxyethyl]-1-methyl-3-(4-benzyloxyphenyl)propylamine

To a stirred solution of 10.0 g of R,R-N-[2-(4-benzyloxyphenyl)-2-hydroxy-1-oxoethyl]-1-methyl-3-(4-benzyloxyphenyl)propylamine in 500 ml of freshly distilled tetrahydrofuran under a nitrogen gas atmosphere was added dropwise over thirty minutes 41 ml of 2 molar borane-dimethyl sulfide complex in tetrahydrofuran. The reaction mixture was stirred at 25°C for twenty hours, and then was heated to reflux and stirred for an additional three hours. After cooling the reaction mixture to 25°C and stirring for another eighteen hours, the excess borane was decomposed by the slow portion-wise addition of 400 ml of methanol. The solvent was then removed from the reaction mixture by evaporation under reduced pressure to provide the product as an oil. The oil so formed was dissolved in 250 ml of hot methanol, and after concentrating the volume to 125 ml, the product began crystallizing out of solution. The crystalline product was collected by filtration and recrystallized twice from methanol to provide 6.65 g of R,R-N-[2-(4-benzyloxyphenyl)-2-hydroxyethyl]-1-methyl-3-(4-benzyloxyphenyl)propylamine. M.P. 119—123.5°C.

The amine so formed was dissolved in methanol and added to a solution of ethereal hydrogen chloride, thereby providing 6.49 g of R, R-N-[2-(4-benzyloxyphenyl)-2-hydroxyethyl]-1-methyl-3-(4-benzyloxyphenyl)propylaminium chloride. M.P. 214.5—216°C. $[\alpha]_D$ −13.4°, $[\alpha]_{365}$ −30.2° (MeOH).

Analysis calc. for $C_{32}H_{36}NO_3Cl$
Theory: C, 74.19; H, 7.00; N, 2.70; Cl, 6.84;
Found: C, 74.20; H, 6.98; N, 2.65; Cl, 6.63.

Example 7

R,R-1-(4-Hydroxyphenyl)-2-[1-methyl-3-(4-hydroxyphenyl)propylamino]ethanol hydrochloride, also named as R,R-N-[2-(4-Hydroxyphenyl)-2-hydroxyethyl]-1-methyl-3-(4-hydroxyphenyl)propylaminium chloride

A mixture of 51.6 g of R,R-N-[2-(4-benzyloxyphenyl)-2-hydroxyethyl]-1-methyl-3-(4-benzyloxyphenyl)-propylaminium chloride and 5.0 g of Raney nickel in 2 liters of ethanol and 2 liters of ethyl acetate was stirred at 25°C for four and one-half hours under a hydrogen atmosphere of 60 psi. The reaction mixture was then filtered to remove the residual Raney nickel, and the filtrate was concentrated to an oil by evaporation of the solvent under reduced pressure, and the oil was crystallized from fresh ethanol and diethyl ether to provide 29.8 g of R,R-N-[2-(4-hydroxyphenyl)-2-hydroxyethyl]-1-methyl-3-(4-hydroxyphenyl)propylaminium chloride. M.P. 176—176.5°C. (dec.) $[\alpha]_D$ −22.7°, $[\alpha]_{365}$ −71.2° (3.7 mg/ml MeOH).

Analysis calc. for $C_{18}H_{24}NO_3Cl$
Theory: C, 63.99; H, 7.16; N, 4.15;
Found: C, 63.70; H, 7.26; N, 4.32.

Example 8

As pointed out above, a preferred embodiment of this invention employs a mixture of all four optical isomers of the compound of Example 7. This racemic mixture can be prepared by the method described above by reaction of dl-4-(benzoyloxy)mandelic acid with dl-1-methyl-3-(4-benzyloxyphenyl)propylamine in the presence of DCC to give racemic 1-(4-benzyloxyphenyl)-2-oxo-2-[1-methyl-3-(4-benzyloxyphenyl)-propylamino]ethanol. Reduction of the latter compounds and subsequent removal of the benzyl groups provides racemic 1-(4-hydroxyphenyl)-2-[1-methyl-3-(4-hydroxyphenyl)propylamino]ethanol. This compound is more preferably prepared by the following procedure.

A solution of 32.8 g (0.2 m) of methyl 2-(4-hydroxyphenyl)ethyl ketone and 42.6 g (0.2 m) of 1-(4-hydroxyphenyl)-2-aminoethanol in 380 ml of ethanol containing 3.8 g of five percent palladium on carbon was stirred for sixteen hours at 60°C under hydrogen at 55 psi. The reaction mixture was then filtered, and the filtrate was diluted by addition of 350 ml of water. The aqueous mixture was concentrated to a volume of about 400 ml, and then washed with dichloromethane. The aqueous mixture was acidified by addition of 50 ml of conc. hydrochloric acid. After standing at room temperature for two hours, the aqueous acid mixture was filtered and the filter cake was washed with 40 ml of ice water and dried at 50°C in vacuum to provide 47 g of 1-(4-hydroxyphenyl-2-[1-methyl-3-(4-hydroxyphenyl)propylamino]ethanol hydrochloride. M.P. 124—129°C.

Analysis calc. for $C_{18}H_{24}NO_3Cl$
Theory: C, 63.99; H, 7.16; N, 4.15; Cl, 10.49;
Found: C, 63.77; H, 6.80; N, 3.91; Cl, 10.68.

$^{13}C$ NMR studies demonstrated the product to be comprised of 51% RR,SS diastereamer and 49% RS,SR diastereomer.

Example 9

Premix for Swine

| Ingredient | % by weight |
|---|---|
| 1-(4-hydroxyphenyl)-2-[1-methyl-3-(4-hydroxyphenyl)propylamino]ethanol hydrochloride | 10 |
| Soybean mill run | 88 |
| Mineral oil | 2 |
| | 100 |

The above ingredients are blended to uniformity to provide a dry flowable premix that can be admixed with a typical animal feed ration at a rate to provide about 20 ppm of active ingredient in the final feed ration. For example, the premix can be added to the following swine grower ration for convenient oral administration of the β-phenethanolamine to swine.

| Ingredient | % by weight | lbs/Ton | kg/t |
|---|---|---|---|
| Corn, yellow, ground | 76.70 | 1534 | 767 |
| Soybean Oil Meal, solvent extracted, dehulled | 19.35 | 387 | 194 |
| Calcium Carbonate | 1.20 | 24 | 12 |
| Dicalcium Phosphate, feed grade | 1.20 | 24 | 12 |
| Salt (sodium chloride) | 0.50 | 10 | 5 |
| Trace mineral premix, AN—03[1] | 0.10 | 2 | 1 |
| Swine Vitamin Premix, SW—03[2] | 0.65 | 13 | 7 |
| Vitamin A Premix, 3M USP units/lb.[3] | 0.05 | 1 | 0.5 |
| Methionine Hydroxy Analogue, 93% | 0.20 | 4 | 2 |
| Selenium Premix[4] | 0.005 | 1 | 0.5 |
| | 100.00 | 2000 | 1000 |

[1]Each Kg of premix contains: 50 g manganese as manganese sulfate; 100 g zinc as zinc carbonate; 50 g iron as ferrous sulfate; 5 g copper as copper oxide; 1.5 g iodine as potassium iodide and 150 g maximum and 130 g minimum calcium as calcium carbonate.

[2]Each Kg of premix contains: 77,151 IU Vitamin $D_2$; 2,205 IU Vitamin E; 411 mg riboflavin; 1,620 mg pantothenic acid; 2,205 mg niacin; 4.4 mg Vitamin $B_{12}$; 441 mg Vitamin K; 19,180 mg choline; 110 mg folic acid; 165 mg pyridoxine; 110 mg thiamine; 22 mg biotin.
[3]Each Kg of premix contains 6,613,800 IU Vitamin A.
[4]Each Kg of premix contains 200 mg of selenium as sodium selenite.

A ten day *in vivo* study was employed to determine the effect of β-phenethanolamines on feed efficiency and rate of growth. In these studies, barrows and gilts weighing approximately 60 kg were maintained in individual pens. Each treatment was replicated six times in randomly assigned animals, with each test animal forming an experimental unit. A group of control animals received a normal swine grower feed ration comprising the following ingredients:

| Ingredient | % by weight |
|---|---|
| Ground yellow corn | 76.70 |
| Soybean oil meal | 19.35 |
| Calcium carbonate | 1.20 |
| Dicalcium phosphate | 1.20 |
| Salt | 0.50 |
| Trace mineral premix | 0.10 |
| Swine Vitamin premix | 0.65 |
| Vitamin A premix, 3M USP units/lb. | 0.05 |
| Methionine Hydroxy analogue, 93% | 0.20 |
| Selenium premix | .05 |
| | 100.00 |

The test animals received the same feed ration plus the test compound. All animals received feed and water *ad libitum*. All animals were weighed on day 1 and again on day 10, and feed consumption was measured by recording all feed issued and any remaining feed on day 10. The results of two series of this 10 day test are given in Table I. In the Table, the column labelled "ADG" is the average daily weight gain in kilograms and pours; "ADF" is the average daily feed consumption (in kilograms and pounds) by the test animals; and F/G is the feed efficiency calculated as ADF divided by ADG.

TABLE I

Growth Promotion and Feed Efficiency

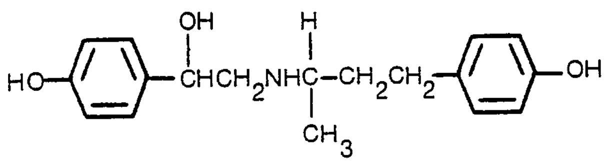

| $R^1$ | dose ppm | ADG | | ADF | | F/G |
|---|---|---|---|---|---|---|
| | | lb | kg | lb | kg | |
| Control | | 1.60 | 0.73 | 4.7 | 2.1 | 2.98 |
| Compound of formula (I) | 20 | 2.19 | 0.99 | 5.0 | 2.3 | 2.33 |

The β-phenethanolamines to be employed in the method of this invention can be administered in combination with other compounds known to have a beneficial effect upon animals. Typical compounds to be co-administered with the β-phenethanolamines include antibiotics, for example any of the tetracyclines, tylosin, penicillins, cephalosporins, polyether antibiotics, glycopeptides, orthosomycins and related compounds commonly administered to swine, poultry, ruminants and the like. A preferred combination to be employed in the present method is an antibiotic such as tylosin or a tetracycline, together with 1-(4-hydroxyphenyl)-2-[1-methyl-3-(4-hydroxyphenyl)propylamino]ethanol. Such combinations will comprise the respective components in a ratio of about 1 to about 2 parts by weight of β-phenethanolamine and about 1 to about 10 parts by weight of the partner component.

A total of 180 crossbred barrows and gilts, averaging approximately 134 pounds starting weight, were used to determine the effect of oral administration of Example 8 at 5 or 20 ppm on growth performance and carcass characteristics of finishing pigs. The pigs were fed a 16% crude protein corn-soy diet during the experiment and were housed in an enclosed commercial type swine building with concrete slatted floors. The effect of treatments on average daily gain (ADG), average daily feed (ADF) and feed/gain are shown in Table II. The treatment effects on the carcass composition of the pigs are presented in Table III.

TABLE II

| Compound | Number Animal | No. Pens | Initial wt. | | ADG | | ADF | | Feed/ Gain |
|---|---|---|---|---|---|---|---|---|---|
| | | | Kg | lb | kg | lb | kg | lb | |
| Control | 19 | 4 | 61 | 134 | 0.68 | 1.51 | 2.40 | 5.30 | 5.30 |
| Example 8 5 ppm | 20 | 4 | 62 | 136 | 0.73 | 1.62 | 2.38 | 5.25 | 3.24 |
| Example 8 20 ppm | 19 | 4 | 62 | 136 | 0.73 | 1.62 | 2.41 | 5.31 | 3.26 |

Animals were slaughtered on a weight constant basis at approximately 230 pounds. Animals were on test an average of 60 days. The control feed and all treated feeds contained Tylosin at 44 ppm (40 g/t).

TABLE III

| Treatment Description | Ave. Live kg/lb | Ave. Yield % | Carc. Leng. cm/in | Leaf-Fat kg/lb | Ave. B.F. cm/in | Loin Fat Depth cm/in | Eye Area Sq. cm/Sq. in | Est. % Musc. | Est. kg/lb Musc. |
|---|---|---|---|---|---|---|---|---|---|
| Control | 103 228 | 70.0 | 82.3 32.4 | 1.22 2.70 | 2.79 1.10 | 2.29 0.90 | 32.58 5.05 | 50.9 | 37.7 83.1 |
| Example 8 5 ppm | 105 231 | 71.2 | 82.0 32.3 | 1.13 2.50 | 2.72 1.07 | 2.16 0.85 | 36.84 5.71 | 53.1 | 40.4 89.1 |
| Example 8 20 ppm | 104 230 | 71.5 | 80.8 31.8 | 1.01 2.23 | 2.62 1.03 | 2.07 0.79 | 37.94 5.88 | 54.1 | 41.2 90.8 |

Key
Ave. = Average
B.F. = Back Fat
Est. = Estimated
Musc. = Muscle Fat

Additional studies have been carried out to demonstrate the anabolic effect of β-phenethanolamines in swine. The effect of the compounds on nitrogen retention in finishing barrows was determined. Nitrogen retention is the difference between nitrogen intake and nitrogen excreted. Increased nitrogen retention is known to be associated with increased anabolic activity, resulting in improved carcass muscling and leanness.

In a typical study, barrows weighing about 172 pounds (78 Kg) were orally administered various doses of 1-(4-hydroxyphenyl)-2-[1-methyl-3-(4-hydroxyphenyl)propylamino]ethanol hydrochloride (Compound A). All animals received water and a constant amount of normal swine feed ration. The results of this study are presented in Table IV, and show that all β-phenethanolamine treatments improved nitrogen retention compared to controls.

TABLE IV

Nitrogen Retention

| Treatment | Animals per treatment | Nitrogen Retained (g/day) |
|---|---|---|
| Control | 6 | 21.0 |
| Compound A (5 g/T) | 3 | 23.6 |
| Compound A (10 g/T) | 3 | 23.9 |
| Compound A (20 g/T) | 3 | 25.0 |

As pointed out above, the method of this invention can be practiced with individual isomers of β-phenethanolamines or with mixtures of isomers and diastereomers. In a study to determine the effect on weight gain and feed efficacy of various mixtures of diastereomers, barrows initially weighing about 177 pounds (80 kg) were fed a normal swine diet plus a β-phenethanolamine at 20 g/T of feedstuff. Each treatment was replicated in twelve individually fed animals. The results are presented in Table V and show that growth performance was improved by both β-phenethanolamine treatments with very little change in feed intake.

TABLE V

| Treatment | Average Daily Feed | | Average Daily Gain | |
|---|---|---|---|---|
| | kg | lb | kg | lb |
| Control | 2.67 | 5.89 | 0.72 | 1.58 |
| 1-(4-hydroxyphenyl)-2-[1-methyl-3-(4-hydroxyphenyl)propylamino]ethanol hydrochloride | 2.69 | 5.94 | 0.98 | 2.15 |
| 57.5% RR,SS | | | | |
| 42.5% RS,SR | | | | |
| 1-(4-hydroxyphenyl)-2-[1-methyl-3-(4-hydroxyphenyl)propylamino]ethanol hydrochloride | 2.66 | 5.86 | 0.88 | 1.95 |
| 47% RR,SS | | | | |
| 53% RS,SR | | | | |

The data in Table V demonstrates that the method of improving feed efficiency and promoting growth can be practiced with any desired mixture of β-phenethanolamine optical isomers.

The efficacy of the β-phenethanolamines described herein also has been demonstrated in poultry. In a typical study, broilers that were twenty-one days old were administered an oral dosing of a β-phenethanolamine in their normal daily feed ration. All animals received the following broiler finisher ration:

| Ungredients | % by weight | lbs/T | kg/t |
|---|---|---|---|
| Ground yellow corn | 66.40 | 1328.00 | 664.00 |
| Animal-vegetable fat | 1.53 | 30.60 | 15.30 |
| Corn Glut. meal (60%) | 4.00 | 80.00 | 40.00 |
| Soybean meal (48%) | 19.19 | 383.80 | 191.90 |
| Fish meal-menhaden | 2.50 | 50.00 | 25.00 |
| Dicalcium phosphate | 1.01 | 34.20 | 17.10 |
| Feather meal-Hydr. | 2.50 | 50.00 | 25.00 |
| Ground limestone | 0.83 | 16.60 | 8.30 |
| Salt | 0.30 | 6.00 | 3.00 |
| Vitamin Premix[1] | 0.50 | 10.00 | 5.00 |
| Trace mineral premix[2] | 0.10 | 2.00 | 1.00 |
| Methionine Hyd. Anal. | 0.15 | 3.00 | 1.50 |
| Lysine HCl | 0.29 | 5.80 | 2.90 |
| | 100.00 | 2000.00 | 1000.00 |

[1]Vitamin premix provides 3000 IU of vitamin A, 900 ICU of vitamin $D_3$, 40 mg of vitamin E, 0.7 mg of vitamin K, 1000 mg of choline, 70 mg of niacin, 4 mg of pantothenic acid, 4 mg of riboflavin, 100 mcg of vitamin $B_{12}$, 100 mcg of biotin and 125 mg of ethoxyquin per kg of complete feed.
[2]Trace mineral premix provides 75 mg of manganese, 50 mg of zinc, 25 mg of iron and 1 mg of iodine per kg of complete feed.

Test animals received with the above ration varying doses of 1-(4-hydroxyphenyl)-2-[1-methyl-3-(4-hydroxyphenyl)propylamino]ethanol hydrochloride (compound A). Each treatment was replicated sixteen times, and the test was terminated when the animals reached fifty-six days of age. The animals were analyzed for weight gain and feed efficiency. The results of this test in broilers is presented in Table VI as mean weight gain and mean feed to gain ratios.

TABLE VI

Growth Performance of Broilers

| Treatment | Dose (g/T | Weight Gain: grams | Weight Gain: % improvement | Feed Efficiency: Feed/Gain Ratio | Feed Efficiency: % change from control |
|---|---|---|---|---|---|
| Control | | 1473 | 0 | 2.336 | 0 |
| Compound A | 10 | 1585 | 7.6 | 2.292 | 1.9 |
| Compound A | 20 | 1613 | 9.5 | 2.298 | 1.6 |
| Compound A | 40 | 1550 | 5.2 | 2.312 | 1.0 |
| Compound A | 80 | 1669 | 13.3 | 2.221 | 4.9 |

The results of this study demonstrate that the β-phenethanolamines described herein are effective in promoting growth and improving feed efficiency in poultry.

The compounds of the invention also have demonstrated efficacy in ruminants. Forty-eight cross-bred wether lambs were employed in a test designed to show the effects of Compound A (1-(4-hydroxyphenyl)-2-[1-methyl-3-(4-hydroxyphenyl)propylamino]ethanol hydrochloride) at varying doses. Sixteen animals were held as controls, while sixteen received 40 ppm of Compound A, and another sixteen received 80 ppm of Compound A. All animals received a normal daily feed ration and water *ad libitum*. Average daily weight gain and average daily feed consumption after twenty-eight days is given below in Table VII. The data demonstrates that a β-phenethanolamine as defined herein is effective in promoting growth and improving feed efficiency in ruminants.

TABLE VII

Growth Performance of Lambs

| Treatment | Dose (ppm) | ADG (kg/lbs) | | ADF (kg/lbs) | | F/G |
|---|---|---|---|---|---|---|
| Control | 0 | 0.188 | 0.414 | 1.669 | 3.68 | 8.89 |
| Compound A | 40 | 0.190 | 0.418 | 1.637 | 3.61 | 8.64 |
| Compound A | 80 | 0.214 | 0.472 | 1.619 | 3.57 | 7.56 |

**Claims**

1. A method for promoting the growth, improving the efficiency of feed utilization or improving the leanness of a domesticated animal, which comprises administering to said animal a compound of the formula (I):

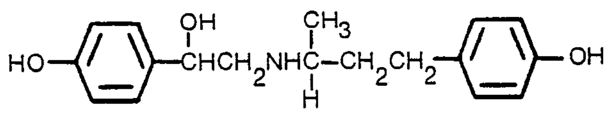 (I)

or an acid addition salt thereof.

2. A method according to claim 1, for growth promotion.

3. A method according to claim 1, for improving the efficiency of feed utilisation.

4. A method according to claim 1, for improving the leanness of a domesticated animal.

5. A method according to any one of claims 1 to 4, wherein the compound of formula (I) is 1-(4-hydroxyphenyl)-2-[1-methyl-3-(4-hydroxyphenyl)propylamino]ethanol, hydrochloride.

6. A method according to any one of claims 1 to 5 which comprises administering a compound of formula (I) to pigs.

7. A method according to any one of claims 1 to 5 which comprises administering a compound of formula (I) to cattle.

8. An animal feed premix comprising a β-phenethanolamine of the formula (I):

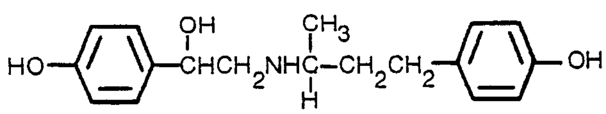 (I)

or an acid addition salt thereof, associated with a suitable carrier therefor.

9. An animal feed premix as claimed in claim 8, wherein the compound of formula (I) is in the form of its hydrochloride.

**Patentansprüche**

1. Verfahren zur Förderung des Wachstums, Verbesserung der Effizienz der Futterverwertung oder Verbesserung der Magerkeit bei einem domestizierten Tier, dadurch gekennzeichnet, daß einem solchen Tier eine Verbindung der Formel (I)

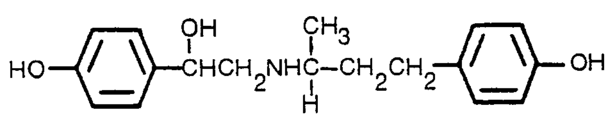 (I)

oder ein Säureadditionssalz hiervon verabreicht wird.

2. Verfahren nach Anspruch 1 zur Förderung des Wachstums.

3. Verfahren nach Anspruch 1 zur Verbesserung der Effizienz der Futterverwertung.

4. Verfahren nach Anspruch 1 zur Verbesserung der Magerkeit eines domestizierten Tieres.

5. Verfahren nach einem der Ansprüche 1 bis 4, dadurch gekennzeichnet, daß die Verbindung der Formel (I) 1-(4-Hydroxyphenyl)-2-[1-methyl-3-(4-hydroxyphenyl)propylamino]ethanol-hydrochlorid ist.

6. Verfahren nach einem der Ansprüche 1 bis 5, dadurch gekennzeichnet, daß eine Verbindung der Formel (I) Schweinen verabreicht wird.

7. Verfahren nach einem der Ansprüche 1 bis 5, dadurch gekennzeichnet, daß eine Verbindung der Formel (I) Rindern verabreicht wird.

8. Tierfuttervormischung aus einem β-Phenethanolamin der Formel (I)

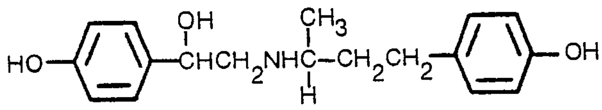 (I)

oder einem Säureadditionssalz hiervon in Kombination mit einem geeigneten Träger hierfür.

9. Tierfuttervormischung nach Anspruch 8, dadurch gekennzeichnet, daß die Verbindung der Formel (I) als Hydrochlorid vorliegt.

**Revendications**

1. Procédé destiné à stimuler la croissance, à améliorer le taux de rendement alimentaire ou à améliorer la teneur en maigre de la viande d'un animal apprivoisé, ce procédé consistant à administrer à cet animal un composé de formule (I):

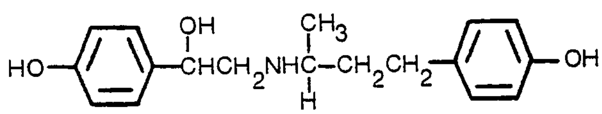 (I)

ou un de ses sels d'addition d'acide.

2. Procédé selon la revendication 1, destiné à stimuler la croissance.

3. Procédé selon la revendication 1, destiné à améliorer le taux de rendement alimentaire.

4. Procédé selon la revendication 1, destiné à améliorer la teneur en maigre de la viande d'un animal apprivoisé.

5. Procédé selon l'une quelconque des revendications 1 à 4, dans lequel le composé de formule (I) est le chlorhydrate du 1-(4-hydroxyphényl)-2-[1-méthyl-3-(4-hydroxyphényl)propylamino]éthanol.

6. Procédé selon l'une quelconque des revendications 1 à 5, ce procédé consistant à administrer un composé de formule (I) à des porcs.

7. Procédé selon l'une quelconque des revendications 1 à 5, ce procédé consistant à administrer un composé de formule (I) à du bétail.

8. Pré-mélange alimentaire pour animaux comprenant une β-phénétanolamine de formule (I):

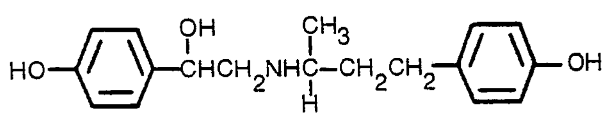 (I)

ou un de ses sels d'addition d'acide, en association avec un support approprié pour ce pré-mélange.

9. Pré-mélange alimentaire pour animaux selon la revendication 8, dans lequel le composé de formule (I) se présente sous forme de son chlorhydrate.